Europäisches Patentamt

European Patent Office

Office européen des brevets

(19)

(11) Publication number: **0 343 388**
**A2**

# (12) EUROPEAN PATENT APPLICATION

(21) Application number: **89107460.1**

(22) Date of filing: **25.04.89**

(51) Int. Cl.4: **C12N 15/00 , C12P 21/02**

The title of the invention has been amended (Guidelines for Examination in the EPO, A-III, 7.3).

The applicant has filed a statement in accordance with Rule 28 (4) EPC (issue of a sample only to an expert). Accession numbers of the deposits: NRRL Y-15851, NRRL B-18114, NRRL B-15867, NRRL B-15868, NRRL B-15869, NRRL B-15890 and NRRL B-18016.

(30) Priority: **25.04.88 US 185219**

(43) Date of publication of application:
**29.11.89 Bulletin 89/48**

(84) Designated Contracting States:
**AT BE CH DE ES FR GB GR IT LI LU NL SE**

(71) Applicant: **PHiLLIPS PETROLEUM COMPANY**
**5th and Keeler**
**Bartlesville Oklahoma 74004(US)**

(72) Inventor: **Thill, Gregory Patrick**
**3284 W. Fox Run Way**
**San Diego, CA 92111(US)**
Inventor: **Davis, Geneva Ruth**
**3083 E. Fox Run Way**
**San Diego, CA 92111(US)**

(74) Representative: **Dost, Wolfgang,**
**Dr.rer.nat.,Dipl.-Chem. et al**
**Patent- & Rechtsanwälte Bardehle .**
**Pagenberg . Dost . Altenburg . Frohwitter .**
**Geissler & Partner Galileiplatz 1 Postfach 86**
**06 20**
**D-8000 München 86(DE)**

(54) **Expression of interferon-gamma in methylotrophic yeasts.**

(57) A process for the production of IFN-γ in methylotrophic yeasts. Also disclosed are novel DNA molecules and methylotrophic yeasts transformed with these molecules.

*FIG. 1*

## EXPRESSION OF INTERFERON-γ IN METYHYLOTROPHIC YEASTS

### Field of Invention

This invention relates to the field of recombinant DNA biotechnology. In one aspect, this invention relates to a process for the expression of interferon-γ (IFN-γ) protein in methylotrophic yeasts. In another aspect the present invention relates to novel DNA molecules coding for IFN-γ and novel yeast strains transformed therewith.

### Background

Interferons (IFNs) are secreted proteins which induce antiviral, cell growth-regulatory, and immunomodulatory actions. Based on antigenicity and biological and chemical properties, interferons have been grouped into three major classes: IFN-α, IFN-β and IFN-γ.

A broad range of biological activities have been attributed to IFN-γ, including blocking cell growth and viral replication, suggesting that IFN-γ may be a potent antitumor agent.

Although this protein has been produced by recombinant means in E. coli, monkey cells and Saccharomyces cerevisiae, each of these systems has significant disadvantages. For example, E. coli produces endotoxins which must be removed by extensive purification, monkey cell lines are expensive to grow compared to yeasts or bacteria, and Saccharomyces cerevisiae has a tendency to spontaneously lose the plasmid(s) which direct the expression of INF-γ.

Thus it would be a significant contribution to the art to develop an alternate process and stable host strains which would produce IFN-γ.

Therefore, it is an object of this invention to provide an alternate process for the production of IFN-γ protein in methylotrophic yeasts.

Yet another object of this invention is to provide novel vectors containing DNA sequences which code for the IFN-γ protein.

A further object of this invention is to provide novel methylotrophic yeasts transformed with a vector or vectors capable of production of IFN-γ.

These and other objects of the invention will become apparent from the disclosure and claims herein.

### Summary of the Invention

In accordance with the present invention, we have discovered a process for the production of IFN-γ comprising transforming a methylotrophic yeast with at least one vector compatible with the host, having an expression cassette containing a structural gene for IFN-γ and culturing the resultant transformants under conditions suitable to obtain the production of IFN-γ.

### Detailed Description of the Figures

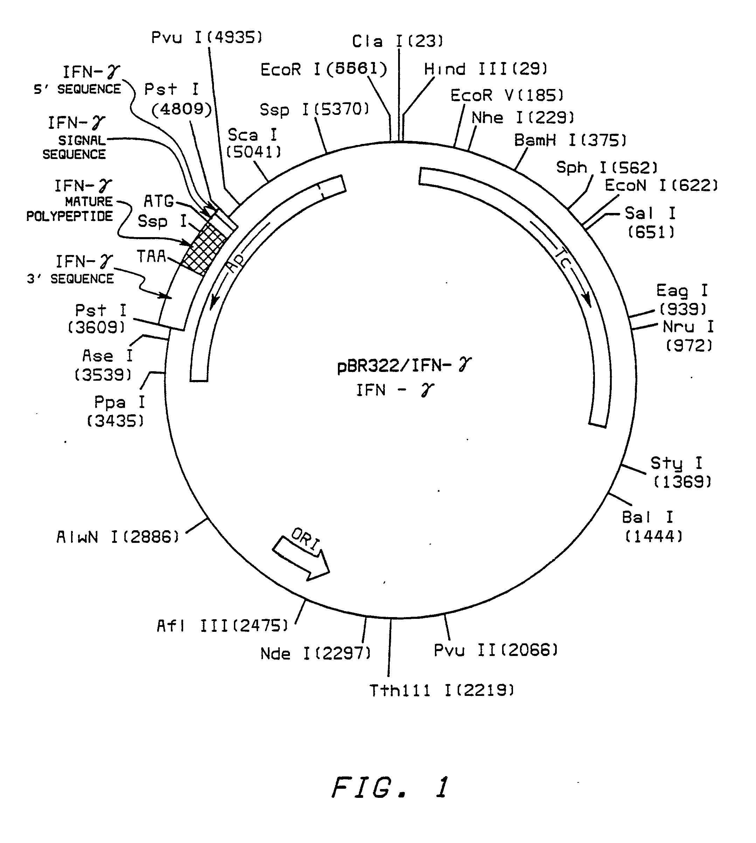

Figure 1 provides a representation of plasmid pBR322/IFN-γ which contains the IFN-γ cDNA inserted at the PstI site of pBR322.

Figure 2 provides a representation of plasmid pAO804 which contains a linear site-specific integrative vector in the fragment clockwise from BglII to BglII. The IFN-γ structural genes INF211 and INFC-2 were inserted in the unique EcoRI site of this vector. < > Brackets indicate that site was destroyed.

Figure 3 provides a flow chart of the construction of vector pINF6, a pAO804 derived plasmid containing a modified IFN-γ gene inserted at the EcoRI site.

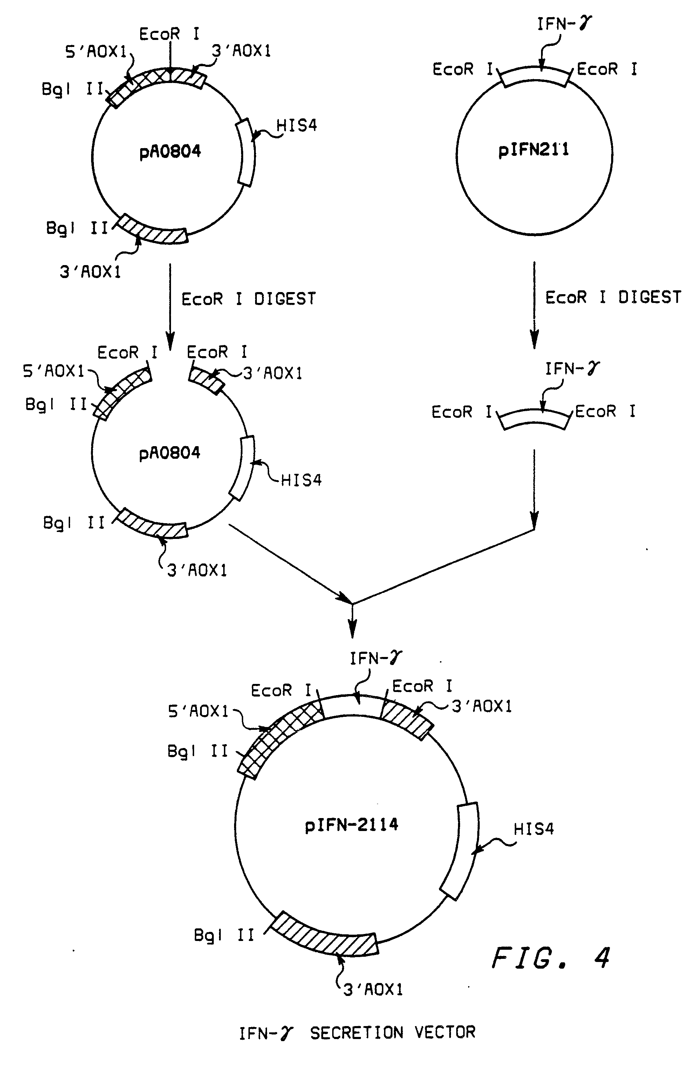

Figure 4 provides a flow chart of the construction of vector pINF-2114, a pAO804 derived plasmid containing the IFN-γ structural gene inserted at the EcoRI site.

## Detailed Description

The IFN-γ is well known in the art and has been sequenced by Gray, Expression of Human Immune Interferon cDNA in E. coli and Monkey Cells, 295 Nature 503 (1982). The structural gene may, therefore, be obtained by reisolating the gene by the method of Gray or synthesized. The structural gene for IFN-γ may be modified or tailor to the vector system and host selected for utilization in expressing the gene by techniques such as M3 mutgenesis.

One nucleotide sequence and amino acid translation of IFN-γ which may be used in the practice of this invention is provided in Table 1. Approximately the first 23 amino acids constitute the signal sequence of the gene.

The pBR322/IFN-γ plasmid is a pBR322 derivative containing the 1200 nucleotide sequence provided in Table 1 inserted in the unique PstI site (3609) of pBR322. This insertion interupts the ampicillin gene of pBR322.

This plasmid may be cultured utilizing suitable E. coli hosts such as MC1061. The plasmid DNA may be recovered from suitable hosts utilizing methods known in the art, such as the method of Birnboim and Doby, Nucleic Acids Research 7:1513 (1979).

The IFN-γ used for the practice of this invention was altered by M13 mutagenesis to accomplish three specific changes of the IFN-γ structural gene. The first mutation created an EcoRI restriction site immediately adjacent to the TAA stop codon (see Table 2 as indicated from approximately nucleotides 500 to 514). The second mutation created an EcoRI site immediately prior to the ATG of the IFN-γ signal sequence (see Table 2 as indicated from approximately nucleotides -9 to 3). The third mutation performed was to delete the signal sequence, and insert an EcoRI restriction site and ATG initiation codon (see Table 2 as indicated from approximately nucleotides 57 to 76).

The M13 mutagenesis is well known in the art and may be performed by any suitable method such as the method of Zoller and Smith, "Oligonucleotide Directed Mutagenesis of DNA Fragments Cloned into M13 Vectors, Methods in Enzymology, 100:468 (1983). M13 vectors and the reagents needed for mutagenesis are available from commercial sources such as New England BioLabs.

Mutagenesis as described above was begun by first inserting the IFN-γ structural gene into the double stranded circular replicative form or RF of the M13 vector (for its ease of use; M3mp19 was selected as the vector of choice). The IFN-γ structural gene in plasmid pBR322/IFN-γ was propogated in E. coli MC1061, and the plasmid DNA was isolated utilizing the method of Birmboim and Doby, supra. The pBR322/IFN-γ plasmid was then digested with PstI. The approximately 1200 base pair PstI fragment containing the IFN-γ structural gene was isolated by gel electrophoresis. This fragment was then inserted into the PstI site of M3mp19. The ligation mixture was then used to transform competent bacterial cells such as JM101 or JM103. Transformants were selected on the basis of a color reaction which indicated the desired fragment had interrupted the β-galactosidase gene and would form clear instead of blue plaques on indicator plates.

The orientation of the insertion may be determined by sequencing, endonuclease digestion and gel electrophoresis, or any other suitable technique. One clone in which the initiator methionine had been inserted close to the M3 universal primer was isolated and used for the first mutagenesis.

Next, a short oligonucleotide was synthesized in vitro consisting of the following nucleotide sequence:
AGAGC-ATCCC-AATAA-GAATT-CTCCT-GCCTG-CAATAT.

The synthetic sequences used in the practice of this invention may be produced by either enzymatic or chemical means. Suitable means include but are not limited to chemical procedures based on phosphotriester or phosphite chemistry.

The single stranded form of M13 with the inserted IFN-γ structural gene was prepared. The synthetic oligonucleotide which is partially complementary to the 3' end between approximately nucleotide 487 to 521 of the IFN-γ structural gene was then annealed to the single stranded M13 vector containing the IFN-γ structural gene. Using the partially complementary synthetic oligonucleotide as a primer, DNA synthesis is carried out in vitro with the Klenow fragment and deoxyoligonucleotide triphosphates at 4°C. The partially complementary synthetic oligonucleotide was then extended around the circular M13 template. The reaction mix was used to transform competent JM101 or JM103 cells. The transformants were screened by transferring the plaques to nitrocellulose and hybridizing with a radioactively labeled oligonucletide such that mutant strands hybridized, while the original template did not. These mutants were then used to prepare template, which was used to transform JM101. These transformants were again screened as before. Positives from the second screening are used to make template for sequencing RF for recovery of the insert. This plasmid was designated pINF.

The M13 isolated above was then subjected to, separately, another mutation to add an EcoRI site 5'

3

immediately adjacent to the ATG start codon of IFN-γ, and a mutation which deleted the signal sequence by creating a EcoRI site and a second start codon at approximately nucleotides 66, 67 and 68 (See Table 2).

To add an EcoRI site 5' immediately adjacent to the ATG start codon another oligonucleotide was synthesized consisting of the following sequence:

TTCTC TCGGA ATTCA TGAAA TATAC.

This sequence was annealed to single stranded pIFN. This oligonucleotide was used as a primer for DNA synthesis as described above. The mutated IFN-γ structural gene was designated INF211, provided in Table 3.

Another mutation of pIFN was performed to delete the signal sequence by creating a EcoRI site and a second start codon at approximately nucleotides 66, 67 and 68. An oligonucleotide was synthesized consisting of the following sequence:

TTGGG-TTCTC-TTGGC-GAATT-CATGC-AGGAC-CCATA-TGTA.

This oligonucleotide was used as a primer for DNA synthesis as described above. This mutated IFN-γ structural gene was designated IFNC-2, as provided in Table 4.

## Table 1

Sequence of gamma-interferon: DNA and protein

```
    -100                    -80                    -60                    -40
CTGAAGATCAGCTATTAGAAGAGAAAGATCAGTTAAGTCCTTTGGACCTGATCAGCTTGATACAAGAACT

          -20                     1                    20
ACTGATTTCAACTTCTTTGGCTTAATTCTCTCGGAAACGATGAAATATACAAGTTATATCTTGGCTTTTC
                                    MetLysTyrThrSerTyrIleLeuAlaPheG

        40                     60                    80                    100
AGCTCTGCATCGTTTTGGGTTCTCTTGGCTGTTACTGCCAGGACCCATATGTAAAAGAAGCAGAAAACCT
lnLeuCysIleValLeuGlySerLeuGlyCysTyrCysGlnAspProTyrValLysGluAlaGluAsnLe

          120                    140                    160
TAAGAAATATTTTAATGCAGGTCATTCAGATGTAGCGGATAATGGAACTCTTTTCTTAGGCATTTTGAAG
uLysLysTyrPheAsnAlaGlyHisSerAspValAlaAspAsnGlyThrLeuPheLeuGlyIleLeuLys

          180                    200                    220                    240
AATTGGAAAGAGGAGAGTGACAGAAAAATAATGCAGAGCCAAATTGTCTCCTTTTACTTCAAACTTTTTA
AsnTrpLysGluGluSerAspArgLysIleMetGlnSerGlnIleValSerPheTyrPheLysLeuPheL

          260                    280                    300
AAAACTTTAAAGATGACCAGAGCATCCAAAAGAGTGTGGAGACCATCAAGGAAGACATGAATGTCAAGTT
ysAsnPheLysAspAspGlnSerIleGlnLysSerValGluThrIleLysGluAspMetAsnValLysPh

          320                    340                    360                    380
TTTCAATAGCAACAAAAAGAAACGAGATGACTTCGAAAAGCTGACTAATTATTCGGTAACTGACTTGAAT
ePheAsnSerAsnLysLysLysArgAspAspPheGluLysLeuThrAsnTyrSerValThrAspLeuAsn

          400                    420                    440
GTCCAACGCAAAGCAATACATGAACTCATCCAAGTGATGGCTGAACTGTCGCCAGCAGCTAAAACAGGGA
ValGlnArgLysAlaIleHisGluLeuIleGlnValMetAlaGluLeuSerProAlaAlaLysThrGlyL

          460                    480                    500                    520
AGCGAAAAAGGAGTCAGATGCTGTTTCAAGGTCGAAGAGCATCCCAATAATGGTTGTCCTGCCTGCAATA
ysArgLysArgSerGlnMetLeuPheGlnGlyArgArgAlaSerGln stop
                                              codon

          540                    560                    580
TTTGAATTTTAAATCTAAATCTATTTATTAATATTTAACATTATTTATATGGGGAATATATTTTTAGACT

          600                    620                    640                    660
CATCAATCAAATAAGTATTTATAATAGCAACTTTTTGTGTAATGAAAATGAATATCTATTAATATATGTA

          680                    700                    720
TTATTTATAATTCCTATATCCTGTGACTGTCTCACTTAATCCTTTGTTTTCTGACTAATTAGGCAAGGCT

          740                    760                    780                    800
ATGTGATTACAAGGCTTTATCTCAGGGGCCAACTAGGCAGCCAACCTAAGCAAGATCCCATGGGTTGTGT
```

## Table 1 (Continued)

```
              820                 840                 860
GTTTATTTCACTTGATGATACAATGAACACTTATAAGTGAAGTGATACTATCCAGTTACTGCCGGTTTGA

              880                 900                 920                 940
AAATATGCCTGCAATCTGAGCCAGTGCTTTAATGGCATGTCAGACAGAACTTGAATGTGTCAGGTGACCC

              960                 980                 1000
TGATGAAAACATAGCATCTCAGGAGATTTCATGCCTGGTGCTTCCAAATATTGTTGACAACTGTGACTGT

    1020                 1040                1060                1080
ACCCAAATGGAAAGTAACTCATTTGTTAAAATTATCAATATCTAATATATATGAATAAAGTGTAAGTTCA

    1100
CAACTAAAAAAAAAAAAAAAAAAAA
```

## Table 2

1st mutation

    Native sequence:  500
                       TAA-TGG-TTG-TCC-TGC-CTG

    Mutated sequence:  TAA-GAA-TTC-TCC-TGC-CTG
    (stop)
                       EcoRI site

2nd mutation

    Native sequence:  -9        1
                       TCG-GAA-ACG-ATG

    Mutated sequence:  TCG-GAA-TTC-ATG
                                (start)
                  EcoRI site

3rd mutation

    Native sequence:  55    60        70
                       CTT-GGC-TGT-TAC-TGC-CAG-GAC-CCA

    Mutated sequence:  CTT-GGC-GAA-TTC-ATG-CAG-GAC-CCA
                             (start)

                  EcoRI site

## Table 3

Sequence of <u>EcoRI</u> fragment containing IFN211: DNA and protein

```
                                                      20
                        GAATTCATGAAATATACAAGTTATATCTTGGCTTTTC
                            MetLysTyrThrSerTyrIleLeuAlaPheG
                              (5' Second Mutagenesis)

      40                  60                  80                 100
AGCTCTGCATCGTTTTGGGTTCTCTTGGCTGTTACTGCCAGGACCCATATGTAAAAGAAGCAGAAAACCT
lnLeuCysIleValLeuGlySerLeuGlyCysTyrCysGlnAspProTyrValLysGluAlaGluAsnLe

          120                 140                 160
TAAGAAATATTTTAATGCAGGTCATTCAGATGTAGCGGATAATGGAACTCTTTTCTTAGGCATTTTGAAG
uLysLysTyrPheAsnAlaGlyHisSerAspValAlaAspAsnGlyThrLeuPheLeuGlyIleLeuLys

      180                 200                 220                 240
AATTGGAAAGAGGAGAGTGACAGAAAAAATAATGCAGAGCCAAATTGTCTCCTTTTACTTCAAACTTTTTA
AsnTrpLysGluGluSerAspArgLysIleMetGlnSerGlnIleValSerPheTyrPheLysLeuPheL

          260                 280                 300
AAAACTTTAAAGATGACCAGAGCATCCAAAAGAGTGTGGAGACCATCAAGGAAGACATGAATGTCAAGTT
ysAsnPheLysAspAspGlnSerIleGlnLysSerValGluThrIleLysGluAspMetAsnValLysPh

          320                 340                 360                 380
TTTCAATAGCAACAAAAAGAAACGAGATGACTTCGAAAAGCTGACTAATTATTCGGTAACTGACTTGAAT
ePheAsnSerAsnLysLysLysArgAspAspPheGluLysLeuThrAsnTyrSerValThrAspLeuAsn

          400                 420                 440
GTCCAACGCAAAGCAATACATGAACTCATCCAAGTGATGGCTGAACTGTCGCCAGCAGCTAAAACAGGGA
ValGlnArgLysAlaIleHisGluLeuIleGlnValMetAlaGluLeuSerProAlaAlaLysThrGlyL

          460                 480                 500
AGCGAAAAAGGAGTCAGATGCTGTTTCAAGGTCGAAGAGCATCCCAATAAGAATT C
ysArgLysArgSerGlnMetLeuPheGlnGlyArgArgAlaSerGln stop
                                                     codon
```

## Table 4

EcoRI fragment containing sequence of IFNC-2: DNA and protein

```
           40                  60                  80                 100
                              GAATTCATGCAGGACCCATATGTAAAAGCAGAAAACCT
                                   MetTyrValLysGluAlaGluAsnLe
                              EcoRI  GLN ASP PRO
```

```
          120               140 ──────────────160
TAAGAAATATTTTAATGCAGGTCATTCAGATGTAGCGGATAATGGAACTCTTTTCTTAGGCATTTTGAAG
uLysLysTyrPheAsnAlaGlyHisSerAspValAlaAspAsnGlyThrLeuPheLeuGlyIleLeuLys
```

```
          180               200               220               240
AATTGGAAAGAGGAGAGTGACAGAAAAAATAATGCAGAGCCAAATTGTCTCCTTTTACTTCAAACTTTTTA
AsnTrpLysGluGluSerAspArgLysIleMetGlnSerGlnIleValSerPheTyrPheLysLeuPheL
```

```
          260               280               300
AAAACTTTAAAGATGACCAGAGCATCCAAAAGAGTGTGGAGACCATCAAGGAAGACATGAATGTCAAGTT
ysAsnPheLysAspAspGlnSerIleGlnLysSerValGluThrIleLysGluAspMetAsnValLysPh
```

```
          320               340               360               380
TTTCAATAGCAACAAAAAGAAACGAGATGACTTCGAAAAGCTGACTAATTATTCGGTAACTGACTTGAAT
ePheAsnSerAsnLysLysLysArgAspAspPheGluLysLeuThrAsnTyrSerValThrAspLeuAsn
```

```
          400               420               440
GTCCAACGCAAAGCAATACATGAACTCATCCAAGTGATGGCTGAACTGTCGCCAGCAGCTAAAACAGGGA
ValGlnArgLysAlaIleHisGluLeuIleGlnValMetAlaGluLeuSerProAlaAlaLysThrGlyL
```

```
          460               480               500
AGCGAAAAAGGAGTCAGATGCTGTTTCAAGGTCGAAGAGCATCCCAATAAGAATTG
ysArgLysArgSerGlnMetLeuPheGlnGlyArgArgAlaSerGln  stop
                                                  codon
```

Culturing the E. coli strain listed above may be accomplished by any suitable means. General techniques for culturing E. coli are already known in the art and any adaptation of these methods to the specific requirements of the strains used herein is well within the abilities of those skilled in the art.

Recovery of plasmid DNA from E. coli can be accomplished by several techniques due to its compact size and closed spherical superhelical form. For example, following the harvest, host cells may be pelleted by centrifugation and then resuspended and lysed. The lysate should be centrifuged to remove cell debris and the supernatant containing DNA retained. A phenol extraction can then be performed to remove most other contaminants from the DNA. The phenol-extracted DNA may then be further treated using a density gradient centrifugation or a gel filtration technique to separate the plasmid DNA from the bacterial DNA. The techniques for achieving the separation alluded to above are well known in the art and numerous methods of performing these techniques are known.

Nuclease digestion of the plasmids may be accomplished by choosing appropriate endonucleases which will cut the selected plasmid in such a way as to facilitate the recovery of the IFN-γ structural gene. The endonucleases used will depend on the plasmid from which the IFN gene is to be excised. For example, the mutated IFN-γ structural genes contained in the M13 plasmids described above may most easily be recovered as a EcoRI fragment.

Gel electrophoresis of DNA may be accomplished using numerous techniques. See P. G. Sealy and E. M. Southern, Gel Electrophoresis of Nucleic Acids - A Practical Approach (D. Rickwood and B. D Hames, eds.) p. 39 (1982). Elution may also be accomplished using numerous techniques appropriate for the gel involved, such as electroelution, diffusion, gel dissolution (agarose gels) or physical extrusion (agarose gels). It is additionally recognized that elution may not be necessary with some gels such as high-quality, low melting temperature agarose.

Once the fragment containing the IFN-γ structural gene or fragments thereof is isolated, additional manipulations may be required before it is inserted in the vector. These manipulations may include, but are not limited to the addition of linkers or blunt-ending the fragment.

Preferred for the practice of the present invention was to recover IFN211 and γ-IFNC-2 from their respective M13 plasmids by EcoRI digestion followed by electrophoresis. The IFN211 IFN-γ structural gene could then be employed in the construction of IFN-γ secretion/expression vectors.

The IFNC-2 IFN-γ structural gene having may then be employed in the construction of IFN-γ cytoplasmic/expression vectors.

Following the isolation of the two IFN-γ structural genes (IFN211 and IFNC-2), each gene was inserted into suitable methylotrophic yeast vector, such as a plasmid. Preferable vectors for the practice of this invention are those compatible with the Pichia genus, most preferably Pichia pastoris.

Plasmids have long been one of the basic elements employed in recombinant DNA technology. Plasmids are circular extrachromosomal double-stranded DNA found in microorganisms. Plasmids have been found to occur in single or multiple copies per cell. Included in plasmid DNA is the information required for plasmid reproduction, i.e. an origin of replication is included for bacterial replication. One or more means of phenotypically selecting the plasmid in transformed cells may also be included in the information encoded in the plasmid. Phenotypic or selection markers, such as antibiotic resistance genes or genes which complement defects in the host biochemical pathways, permit clones of the host cells which have been transformed to be recognized, selected, and maintained.

To express the IFN-γ structural genes in methylotrophic yeast, each gene must be operably linked to a 5′ regulatory region and 3′ termination sequence, which forms the expression cassette that will be inserted into the host via a vector.

The following terms are defined herein for the purpose of clarification.

Operably linked--refers to a juxtaposition wherein the components are configured so as to perform their function.

Regulatory region--DNA sequences which respond to various stimuli and affect the rate of mRNA transcription.

3′ Termination sequence--sequences 3′ to the stop codon which function to stabilize the mRNA such as sequences which elicit polyadenylation.

"Pichia compatible" refers to DNA sequences which will perform their normal function in Pichia such as regulatory regions and 3′ termination sequences derived from Pichia.

Preferred for the practice of the present invention are integrative vectors, such as the linear site-specific integrative vector of Cregg, as described in European Application Serial Number 86114700.7. Such vectors comprise a serially arranged sequence of at least 1) a first insertable DNA fragment; 2) a selectable marker gene; and 3) a second insertable DNA fragment.

The first and second insertable DNA fragments are each at least about 200 nucleotides in length and have nucleotide sequences which are homologous to portions of the genomic DNA of species in which they are to be employed. The various components of the integrative vector are serially arranged forming a linear fragment of DNA such that the expression cassette and the selectable marker gene are positioned between the 3′ end of the first insertable DNA fragment and the 5′ end of the second insertable DNA fragment. The first and second insertable DNA fragments are oriented with respect to one another in the serially arranged linear fragment as they are so oriented in the parent genome.

Nucleotide sequences useful as the first and second insertable DNA fragments are nucleotide sequences which are homologous with separate portions of the native genomic site at which genomic modification is to occur. Thus, for example, if genomic modification is to occur at the locus of the alcohol oxidase gene, the first and second insertable DNA fragments employed will be sequences homologous with separate portions of the alcohol oxidase gene locus. For genomic modification in accordance with the present invention to occur, the two insertable DNA fragments must be oriented with respect to one another in the linear fragment in the same relative orientation as they exist in the parent genome. Examples of nucleotide sequences which could be used as first and second insertable DNA fragments are nucleotide sequences selected from the group consisting of the alcohol oxidase (AOX1) gene, dihydroxyacetone synthase (DHAS) gene, p40 gene and HIS4 gene. The AOX1 gene DHAS gene, p40 gene and HIS4 gene

9

are contained in copending application serial number 780,102 incorporated herein by reference.

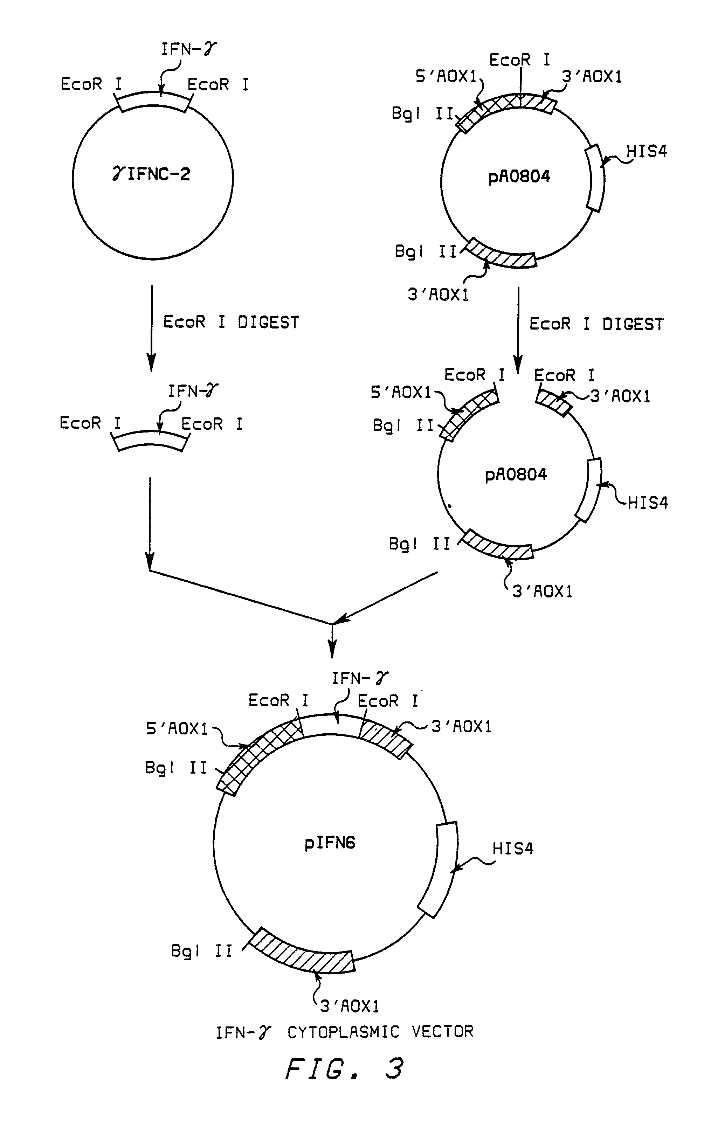

The first insertable DNA fragment may contain an operable regulatory region which may comprise the regulatory region utilized in the expression cassette. The use of the first insertable DNA fragment as the regulatory region for an expression cassette is a preferred embodiment of this invention. Figure 3 provides a diagram of a vector utilizing the first insertable DNA fragment as a regulatory region for a cassette.

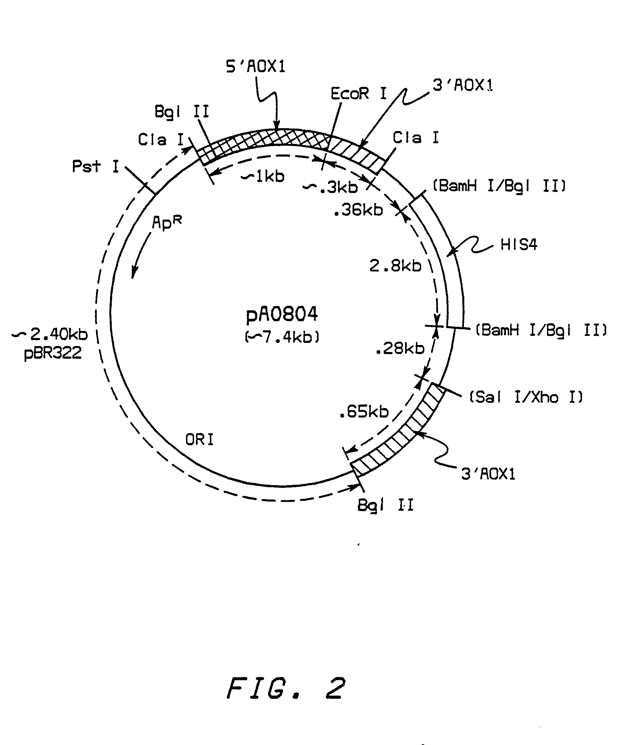

Optionally as shown in Figure 2 an insertion site or sites and a 3′ termination sequence may be placed immediately 3′ to the first insertable DNA fragment. This conformation of the linear site-specific integrative vector has the additional advantage of providing a ready site for insertion of a structural gene without necessitating the addition of a compatible 3′ termination sequence.

It is also necessary to include at least one selectable marker gene in the DNA used to transform the host strain. This facilitates selection and isolation of those organisms which have incorporated the transforming DNA. The marker gene confers a phenotypic trait to the transformed organism which the host did not have, e.g., restoration of the ability to produce a specific amino acid where the untransformed host strain has a defect in the specific amino acid biosynthetic pathway or resistance to antibiotics and the like.

Exemplary selectable marker genes may be selected from the group consisting of the HIS4 gene and the ARG4 gene from Pichia pastoris and Saccharomyces cerevisiae, the invertase gene (SUC2) from Saccharomyces cerevisiae, or the G418 phosphotransferase gene from the E. coli transposable elements Tn601 or Tn903.

Those of skill in the art recognize that additional DNA sequences can also be incorporated into the vectors employed in the practice of the present invention, such as for example, bacterial plasmid DNA, bacteriophage DNA, and synthetic sequences such as polylinkers and the like. Such sequences facilitate the insertion of a gene, the amplification and the maintenance of these vectors in bacterial hosts.

If the first insertable DNA fragment does not contain a regulatory region, a suitable regulatory region will need to be inserted operably linked to the structural gene, in order to provide an operable expression cassette. Similarly if no 3′ termination sequence is provided at the insertion site to complete the expression cassette, a 3′ termination sequence will have to be operably linked to the structural gene to be inserted.

Those skilled in the art are aware of numerous regulatory regions which have been characterized and could be employed in conjunction with methylotrophic yeasts. Exemplary regulatory regions include but are not limited to yeast regulatory regions selected from the group consisting of acid phosphatase, galactokinase, alcohol dehydrogenase, cytochrome c, alpha-mating factor and glyceraldehyde 3-phosphate dehydrogenase regulatory regions isolated from Saccharomyces cerevisiae; the primary alcohol oxidase (AOX1), dihydroxyacetone synthase (DHAS), the p40 regulatory regions, and the HIS4 regulatory region derived from Pichia pastoris and the like. Presently preferred regulatory regions employed in the practice of the present invention are those characterized by their ability to respond to methanol-containing media, such regulatory regions selected from the group consisting of AOX1, DHAS1, and p40 disclosed in EP-A-0183071.

The most preferred regulatory region for the practice of this invention is the AOX1 regulatory region.

3′ termination sequences may be utilized in the expression cassette or be part of the vector as discussed above. 3′ termination sequences may function to terminate, polyadenylate and/or stabilize the messenger RNA coded for by the structural gene when operably linked to a gene. A few examples of illustrative sources for 3′ termination sequences for the practice of this invention include but are not limited to the Saccharomyces cerevisiae, Hansenula polymorpha, and Pichia 3′ termination sequences. Preferred are those derived from Pichia pastoris such as those selected from the group consisting of the 3′ termination sequences of AOX1 gene, DHAS gene, p40 gene and HIS4 gene. And particularly preferred is the 3′ termination sequence of the AOX1 gene.

For the practice of the current invention it is currently preferred to use linear transformation vectors such as the BglII fragment of the vector shown in Figure 2.

The insertion of the IFN211 or IFNC-2 structural gene into suitable vectors may be accomplished by any suitable technique which cleaves the chosen vector at an appropriate site or sites and results in at least one operable expression cassette containing the IFN211 or IFNC-2 structural gene being present in the vector.

Ligation of IFN211 or IFNC-2 structural gene may be accomplished by any appropriate ligation technique such as utilizing T4 DNA ligase.

The initial selection, propagation, and optional amplification of the ligation mixture of an IFN211 or IFNC-2 structural gene within a vector is preferably performed by transforming the mixture into a bacterial host such as E. coli. Suitable transformation techniques for E. coli are well known in the art. Additionally, selection markers and bacterial origins of replication necessary for the maintenance of a vector in a bacterial host are also well known in the art.

The isolation and/or purification of the desired plasmid containing the IFN211 or IFNC-2 structural gene in an expression system may be accomplished by any suitable means for the separation of plasmid DNA from the host DNA.

Similarly the vectors formed by ligation may be tested preferably after propagation to verify the presence of the desired structural gene and its operable linkage to a regulatory region and a 3' termination sequence. This may be accomplished by a variety of techniques including but not limited to endonuclease digestion, gel electrophoresis, or endonuclease digestion-Southern hybridization.

Transformation of circular plasmids or linear vectors into yeast hosts may be accomplished by suitable transformation techniques including but not limited to those taught by Hinnen et al, Proc. Natl. Acad. Sci. 75, (1978) 1929; Ito et al, J. Bacteriol 153, (1983) 163; Cregg et al Mol. Cell Biol. 5 (1985) pg. 3376; or Sreekrishna et al, Gene, 59 (1987) pg. 115. Preferable for the practice of this invention is the transformation technique of Cregg. It is desirable for the practice of this invention to utilize an excess of linear vectors and to identify strains having multiple insertions by Southern hybridization.

Although site-specific linear transformation vector may preferentially insert in one site of the host genome, it is recognized that in the practice of the current invention other sites may be integrated into with a lower frequency. Additionally it is recognized that within homologous DNA regions, each vector may insert in a slightly different placement. Similarly, different multiple insertions may also occur.

The yeast host for transformation may be any suitable methylotrophic yeast. Methylotrophic yeasts include but are not limited to yeast capable of growth on methanol selected from the group consisting of Hansenula, Candida, Kloeckera, Pichia, Saccharomyces, Torulopsis and Rhodotorula. A list of specific species which are exemplary of this class of yeasts may be found in C. Anthony, The Biochemistry of Methylotrophs, 269 (1982). Presently preferred are methylotrophic yeasts of the genus Pichia. Particularly preferred is Pichia pastoris. Auxotrophic methylotrophic yeasts are also advantageous to the practice of this invention for their ease of selection such as the auxotrophic Pichia pastoris GS115 (NRRL Y-15851). It is recognized that wild type methylotrophic yeast strains may be employed with equal success if a suitable transforming marker gene is selected, such as the use of SUC2 to transform Pichia pastoris to a strain capable of growth on sucrose, or if an antibiotic resistance marker is employed, such as G418.

Transformed methylotrophic yeast cells can be selected for by using appropriate techniques including but not limited to culturing previously auxotrophic cells after transformation in the absence of a biochemical product required (due to the cell's auxotrophy), selection by the detection of a new phenotype ("methanol slow"), or culturing in the presence of an antibiotic which is toxic to the yeast in the absence of a resistance gene contained in the transformant.

Isolated transformed methylotrophic yeast cells are cultured by appropriate fermentation techniques such as shake flask fermentation, high density fermentation, or the techniques disclosed by Cregg et al., in High-Level Expression and Efficient Assembly of Hepatitis B Surface Antigen in the Methylotrophic Yeast, Pichia Pastoris, 5 Bio/Technology 479 (1987). ·

Gene expression may be accomplished by methods appropriate to the regulatory region employed. Preferably, if methanol-responsive regulatory regions are utilized, the IFN gene expression may be accomplished by exposing the transformed cells, in a nutrient media, to an appropriate alcohol for the AOX1 regulatory region such as methanol.

The IFN-γ protein may be recovered from strains expressing the cytoplasmic form of the γ-IFN structural gene, in a crude form by lysing transformed cells which have been induced for a sufficient period, using standard techniques such as bead milling, followed by centrifugation sufficient to remove cellular debris. Alternatively, γ-IFN protein may be recovered from the media in which strains expressing the secreted form of the γ-IFN structural gene are grown. Those of skill in the art are aware of numerous methods available for the extraction of a heterologous protein from unicellular host organisms which could be substituted for the general extraction technique above or for further purification. Examples of other suitable techniques may also be found in Example VIII.

The following non-limiting examples are provided to further illustrate the practice of this invention.

## Examples

General information pertinent to the Examples:

Strains

Pichia pastoris GS115 (his4) [NRRL Y-15851] was the host yeast strain used in these examples.

E. coli JM103 delta (lac pro) thi rpsl (strA) supE endA sbcB hsdR.

E. coli K12 MC1061 NRRL-18016 (F⁻, araD139 delta (lac 1POZY)x74 galk galu hsr hsm(+) rpsL delta (araABOIC leu)7697.

Buffers, and Solutions, and Media

The buffers and solutions employed in the following examples have the compositions given below:

dH₂0                    deionized H₂0 that has been treated with a milli-Q (Millipore) reagent water system.

1M Tris buffer          121.1 g Tris base in 800 mL of H₂0; adjust pH to the desired value by adding concentrated (35%) aqueous HCl; allow solution to cool to room temperature before final pH adjustment, dilute to a final volume of 1L.

TE buffer               1.0 mM EDTA in 0.01 M (pH 8.0) Tris buffer

SED                     1 M sorbitol
                        25 mM EDTA
                        50 mM DTT
                        --adjust to pH 8

SCE                     9.1g sorbitol
                        1.47g Sodium citrate
                        0.168g EDTA
                        --pH to 5.8 with HCl in 50 ml
                        dH₂0 and autoclave

CaS                     1 M sorbitol
                        10 mM CaCl₂
                        --filter sterilize

| | |
|---|---|
| PEG Solution | 20% polyethylene glycol-3350<br>10 mM $CaCl_2$<br>10 mM Tris-HCl (pH 7.4)<br>--filter sterilize |
| Solution A | 0.2M Tris-HCl (pH 7.5)<br>0.1M $MgCl_2$<br>0.5M NaCl<br>0.01M dithiothreitol (DTT) |
| Solution B | 0.2M Tris-HCl (pH 7.5)<br>0.1M $MgCl_2$<br>0.1M DTT |
| Solution C (keep on ice) | 4µl solution B<br>4µl 10mM dATP<br>4µl 10mM dTTP<br>4µl 10mM dGTP<br>4µl 10mM dCTP<br>4µl 10mM ATP<br>6µl $T_4$ ligase (2U/µl)<br>12µl $H_2O$<br>Recipe for Solution C was modified from Zoller & Smith |
| 20X SSPE | 4.4g NaOH<br>7.4g $Na_2EDTA$<br>27.6g $NaH_2PO_4 \cdot H_2O$<br>210g NaCl<br>--pH adjusted to 7.5-8.0 with NaOH<br>--$H_2O$ to 1 liter |
| 50X Denhardt's | 5g Ficoll 400<br>5g Polyvinylpyrolidine<br>5g BSA Fraction V<br>$H_2O$ to 500 ml |
| 20X SSC | 175.3g NaCl<br>88.2g sodium citrate<br>--pH to 7.0 with NaOH<br>--$H_2O$ to 1 liter |
| medium composition (7.0-liter) | 480g glycerol<br>40mg biotin<br>134ml $H_3PO_4$ (85%)<br>5.8g $CaSO_4 \cdot 2H_2O$<br>92g $K_2SO_4$<br>75g $MgSO_4 \cdot 7H_2O$<br>21g KOH |

| IM1 Trace Salts Solution, 1 liter | 0.06g Cupric Sulfate·5H$_2$O |
|---|---|
| | 0.08g Potassium Iodide |
| | 0.30g Manganese Sulfate·H$_2$O |
| | 0.20g Sodium Molybdate |
| | 0.02g Boric Acid |
| | 2.00g Zinc Sulfate·H$_2$O |
| | 4.8g Ferric Chloride·H$_2$O |
| | 5.00ml/liter Sulfuric Acid |
| LB Broth, 1 liter | 5.0g yeast extract |
| | 10.0g tryptone |
| | 5.0g NaCl |
| 10X Transfer Buffer | 96.8g Trizma Base |
| | 9.74g glycine |
| | water to 1 liter |
| Transfer Buffer for Tank | 500mls 10X Transfer Buffer |
| | 1000 mls methanol |
| | 3500 mls water |
| Western Buffer - for 1 liter | 2.5g gelatin put in solution by microwaving first in 100 mls water |
| | 100mls 10X PBS |
| | 1ml 50% Tween-20 |
| | 4mls 5% sodium azide |
| | dH$_2$O to 1 liter |

Example I

Construction of γIFNC-2 and pIFN211

A. Mutagenesis of IFN-γ Insert

DNA encoding IFN-γ was obtained from pBR322/IFN-γ by PstI digestion as described in Example II and a 1.2 kb fragment was recovered by a 0.8% preparative agarose gel electrophoresis. The DNA was mutagenized by the following procedure to make the following changes: 1) add an EcoRI restriction site immediately adjacent to the TAA stop codon in the IFN-γ cDNA, 2) add an EcoRI restriction site immediately prior to the ATG of the IFN-γ signal sequence (for the secretion vectors), and 3) delete the signal sequence (for the cytoplasmic expression vectors).

The oligonucleotides employed in the mutagenesis were:

1) 3' mutagenesis to add EcoRI

AGAGC-ATCCC-AATAA-GAATT-CTCCT-GCCTG-CAATAT

2) 5' mutagenesis to add EcoRI prior to signal (secretion)

TTCTC-TCGGA-ATTCA-TGAAA-TATAC

3) 5' mutagenesis to delete signal and add EcoRI (cytoplasmic)

TTGGG-TTCTC-TTGGC-GAATT-CATGC-AGGAC-CCATA-TGTA

and were synthesized using an Applied Biosystems DNA Synthesizer, Model 380A using cyanoethylphosphoramidite chemistry.

Double strained m13mp18 was digested with PstI and dephosphorylated as described in Example II and ligated with the previously isolated 1.2 kb PstI fragment.

The ligation mixture was transformed into competent JM103 cells (competent JM103 were prepared as described in Example II for MC1061 cells). The mixture was then plated on LB media containing 1PTG and X-gal and the plates screened for clear plaques.

A. A large scale miniprep was performed on positive plaques which had been incubated for approximately 7 hours in 2 mls of L media. 25 mls of LB media was inoculated with 250 $\mu$l of freshly grown JM103 cells. The culture was grown for 1 hour and inoculated with 100 $\mu$l of the 7 hour old plaque culture. The culture was then grown overnight. The culture was then centrifuged twice at 10,000 rpms for 10 minutes on a Sorvall RC-5B rotor SS34 to clear the supernatant. 3.5 ml of 20% PEG/2.5M NaCl was added to the culture and it was incubated for 5 hours at 4°C. The culture was then centrifuged again as above for 10 minutes. The supernatant was discarded and the pellet was resuspended in 2 mls of TE buffer. The pellet was then extracted with phenol, equilibrated with TE, extracted with phenol/chloroform extracted twice with CHCl₃ and once with ether. 8M LiCl was added to attain a final concentration of 0.8M. 3 volumes of ethanol were added and the solution left overnight at 20°C to precipitate the DNA present. The solution was next centrifuged for 10,000 rpms for 10 minutes as previously described and rinsed with 70% ethanol. The precipitate was resuspended in 150 $\mu$l of 10mM Tris (pH 7.4).

B. One pmole of M13 recombinant template was mixed with 20 pmole of oligonucleotide 1 (for 3' mutagenesis to create an EcoRI), 1 $\mu$l of solution A and dH₂0 was added to give a final volume 10 $\mu$l. The sample was incubated at $\overline{65}$°C for 5 minutes, and the temperature was then reduced to 37°C for 30 minutes.

C. The following was then added to the sample:

| Solution B | 1 $\mu$l |
|---|---|
| 10 mM dATP | 1 $\mu$l |
| 10 mM dCTP | 1 $\mu$l |
| 10 mM dGTP | 1 $\mu$l |
| 10 mM dTTP | 1 $\mu$l |
| 5u/$\mu$l klenow | 2 $\mu$l |
| dH₂O | 3 $\mu$l |
| | $\overline{\overline{20 \, \mu l}}$ |

and allowed to incubate at 15°C for at least 4-6 hours.

D. The sample was then diluted 1:40 with dH₂O. 5 $\mu$l was used to transform 6 tubes of competent JM103 cells (200 $\mu$l each). The transformed JM103 cells were plated on rich media in a soft agar overlay.

E. The positive plaques were then screened for by filter hybridization.

A hybridization probe of 15 pmole of complementary oligonucleotide in a total volume of 25 $\mu$l total volume was heated to 65°C for 10 minutes. 3 $\mu$l 10X kinase buffer (Maniatis), 1 $\mu$l γ-ATP and 1 $\mu$l polynucleotide kinase (100$\mu$/$\mu$l) were added to the sample. The sample was incubated for 1 hour at 37°C and run through G-50 fine sephadex. The first peak off the column was collected.

Nitrocellulose filters were prepared for hybridization with the above probe by placing and orienting the filters on the transformation plates for 5-10 minutes. The filters were then removed from the plates and floated on a denaturing solution (1.5M NaCl, 0.5N NaOH) for 3 minutes with the backside on top of the solution. The filters were then submerged in the denaturing solution for 5 minutes. The nitrocellulose filters were then transferred to a neutralizing solution (1M Tris•/HCl, pH 8; 1.5M NaCl) for 5 minutes. The neutralized filter was then transferred to 2XSSC (1XSSC is 150 mM NaCl, 15mM NaCitrate) for 5 minutes. The filter was then air dried and baked for 1 hour at 80°C under a vacuum. The filters were prehybridized for 1 hour at 65°C in a sealed plastic bag containing 5 ml of hybridization buffer filter, 10X Denhardts (1X Denhardts is 0.02% Ficoll, 0.02% polyvinyl pyrollidone, 0.02% bovine serum albumin) 0.5% SDS and 5XSSPE. Replace the hybridization buffer with 5ml/filter of fresh hybridization buffer. The radioactive complementary oligonucleotide previously prepared was first incubated at 65°C for 5 minutes, and then enough probe added to the fresh hybridization buffer containing the filter to give 1X10⁶ cpm/ml. Hybridization was performed at 5°C below the calculated melting temperature of the probe for 4 hours.

The filters were then washed three times for 10 minutes each with 6X SSC at room temperature. The filters were finally washed one time with 6XSSC at the hybridization temperature. The filters were placed on a 3 MM Watman paper to dry, and then exposed to film (marked for orientation) overnight.

Three positive plaques were each picked and grown separately in 2 mls of LB broth at 37°C for 5 hours.

F. Mini template preps were performed on each of these positive plaques.

One ml of the plaque culture was transferred into an eppendorf tube and centrifuged for 5 minutes in a Eppendorf Model 5414 Centrifuge. 800 μl of the supernatant was recovered and 200 μl of 20% PEG 2.5M NaCl added thereto. The supernatant was incubated at room temperature for 10 minutes. The supernatant was centrifuged for 10 minutes in the Eppendorf centrifuge previously used. The supernatant was removed by aspiration and the pellet formed by centrifuging was redissolved in 200 μl TE (10 mM Tris, pH 7.4; 1mM EDTA). The redissolved pellet was then phenol/chloroform extracted and the template DNA in the upper aqueous phase was precipitated by the addition of a LiCl solution until a 0.8M concentration was reached. 2½-3 volumes of ethanol and precipitated on dry ice for 5 minutes. The precipitate was centrifuged for 10 minutes in the previously mentioned Eppendorf centrifuge. The final volume was brought up to 150 μl TE.

G. 200 μl of competent JM103 cells were transformed with the recovered DNA. 1 μl and 1 μl of a 1/10 dilution of the isolated phage DNA was used in the transformation. H. The transformation mixture was plated and plaques and screened with oligonucleotides as previously described in step E.

I. A large scale miniprep was preformed on positive plaques which had been incubated for approximately 7 hours in 2 mls of L media. 25 mls of LB media was inoculated with 250 μl of freshly grown JM103 cells. The culture was grown for 1 hour and inoculated with 100 μl of the 7 hour old plaque culture. The culture was then grown overnight. The culture was then centrifuged twice at 10,000 rpms for 10 minutes on a Sorvall RC-5B rotor SS34 to clear the supernatant. 3.5 ml of 20% PEG/2.5M NaCl was added to the culture and it was incubated for 5 hours at 4°C. The culture was then centrifuged again as above for 10 minutes. The supernatant was discarded and the pellet was resuspended in 2 mls of TE buffer. The pellet was then extracted with phenol, equilibrated with TE, extracted with phenol/chloroform extracted twice with $CHCl_3$ and once with ether. 8M LiCl was added to attain a final concentration of 0.8M. 3 volumes of ethanol were added and the solution left overnight to precipitate the DNA present. The solution was next centrifuged for 10,000 rpms for 10 minutes as previously described and rinsed with 70% ethanol. The precipitate was resuspended in 150 μl of 10 mM Tris (pH 7.4).

J. The positive plaques were then sequenced by dideoxy sequencing to find the M13 constructs with the correct mutations. One of the correct M13 constructs was designated pIFN.

K. Repeat steps B-J using pIFN as template and the second oligonucleotide as a primer for 5' mutagenesis. The correct mutation was designated pIFN211.

L. Repeat steps B-J using the pIFN as template and the third oligonucleotide as a primer for 5' mutagenesis. The correct mutation was designated γIFNC-2.

M. Recover RF DNA of pIFNC-2 and pIFN211 using the alkaline lysis method of Maniatis.


Example II


Construction of IFN-γ Expression Vectors


pAO804 is available in an E. coli host from the Northern Regional Research Center of the United States Department of Agriculture, Peoria, Illinois, accession number B-18114. pAO804 is recovered by isolating the plasmid DNA, digesting with EcoRI, gel electrophoresing to recover the ~7.5 kb fragment, which is linear pAO804 cut at its unique EcoRI site.

pAO804 is a vector capable of site-specific disruption of the P. pastoris AOX1 locus. It contains the following elements: the AOX1 promoter and transcription terminator separated by a unique EcoRI cloning site; the wild-type Pichia HIS4 gene; a genomic segment of DNA from the 3' end of the AOX1 locus downstream of the transcription terminator; sequences necessary for selection and replication in a bacterial host. The components are arranged such that a BgIII restriction digest of the plasmid releases a DNA fragment containing the expression cassette and selective marker whose ends are homologous to a continuous portion of the genome, the AOX1 locus, and can be stably inserted into the chromosome during transformation.

A vector containing the gene coding for the cytoplasmic production of IFN-γ was constructed from pAO804 and γIFNC-2. pAO804 was digested with EcoRI and the ends were dephosphorylated by treatment with alkaline phosphatase (1 U enzyme at 37°C for 1 hr. in 50mM Tris•Cl, pH 9.0, 1mM $MgCl_2$, 100 mM $ZnCl_2$, 1mM spermidine). γIFNC-2 was also digested with EcoRI, and a 445 bp fragment encoding IFN-γ was released. This fragment was purified using 0.8% preparative agarose gel electrophoresis. 50ng of the fragment was ligated to 5ng of pAO804 by incubation at 23°C for 1 hr. in 66mM Tris•Cl, pH 7.4, 5mM $MgCl_2$, 5mM dithiothreitol, 1mM ATP, with 1 Weiss Unit of T4 ligase in a 10μl reaction volume. The ligation reaction was used to transform competent MC1061 cells to ampicillin resistance.

MC1061 was rendered competent for transformation in the following manner. A mid-log culture (50 ml) of E. coli MC1061 was harvested by centrifugaiton in IEC DPR 600 Clinical centrifuge at 3,000 rpm 5 min. at 4°C and washed in 10 mM NaCl. The culture was resuspended in 25 ml of 50 mM CaCl₂ for 30 minutes at 0°C. The cells were centrifuged as above and resuspended in 2 ml of 50mM CaCl₂. For transformation, the ligation reaction was added to 200 µl of the competent cell suspension and incubated at 0°C on ice for 15 minutes, heat shocked at 37°C for 5 minutes and incubated at 23°C for 5 minutes. The cells were plated directly onto LB agar plates containing 50 µg/ml ampicillin. The plates were incubated at 37°C for 10-16 hours. The resulting colonies were Amp$^R$. The resistant colonies were harvested and characterized by restriction digestion. Cells were grown in 5 ml of L-broth containing 50 µg/ml ampicillin for 5 hr. at 37°C and DNA was prepared by the method of Birnboim and Doly [Nucleic Acids Research 7: 1513 (1979)]. The minipreps displaying 1800 and 1315 bp fragments upon SspI digestion were chosen and designated pIFN6.

For construction of the IFN-γ secretion vector, the 508 bp fragment from pIFN211 was isolated and inserted into pAO804 as described above. Plasmid DNA was isolated from AMP$^R$ colonies and digested with SspI. A clone displaying 1800 and 1375 bp fragments was designated pIFN-2114.

## Example III

## Transformation of Pichia pastoris

Pichia pastoris strains containing the vectors described in Example II were generated in the following manner. Methanol utilization deficient (Mut⁻) and wild type methanol utilization (Mut⁺) strains were developed.

Pichia pastoris strain GS115 (his4; NRRL Y-15851) was transformed with 5 µg of a BglII digest of pIFN6 using the spheroplast transformation technique described by Cregg et al., Bio/Technology 5:479-485 (1987). Transformants were regenerated on minmal media and screened for the appropriate methanol utilization deficient phenotype in the following manner. Transformants were pooled by scraping the surface of the plate in the presence of sterile distilled water and sonicated at low output for 15 seconds. They were subsequently diluted to an $A_{600} = 0.1$ and plated at dilutions of $10^{-3}$ and $10^{-4}$ in duplicate onto minimal plates containing glycerol as the carbon source, and incubated at 30°C for 2-3 days. They were then replica-plated onto minimal plates to which 100 µl of methanol was added in the vapor phase. After a 24-hour incubation at 30°C, it was apparent that 4% of the transformants were growing more slowly on methanol than the rest of the transformants. These strains were designated G-GIF000C.

The secretion plasmid pIFN-2114 was transformed into Pichia pastoris GS115 cells in an identical manner. These strains were designed G-GIF211S.

The faster growing Mut⁺ cytoplasmic IFN-γ Pichia strains were developed by transforming GS115 with 5 µg of circular pIFN6; they were called G+GIF000C. The Mut⁺ secretion strains were developed by transforming GS115 with 5 µg of circular pIFN-2114; they were called G+GIF211S.

## Example IV

## Yeast DNA Miniprep

$10^4$ cells/ml were seeded in 5ml YPD at 30°C overnight and then pelleted using a Damon IEC DPR600 clinical centrifuge at 3,000 rpm for 5 minutes. The pellet was resuspended in 0.5ml of 1M sorbitol, 0.1ml 0.5M EDTA, pH 8 and the sample transferred to a 1.5ml microfuge tube. 0.02ml of 2.5 mg/ml Zymolyase 100,000 (Miles Laboratories) was added, and the sample was incubated at 37°C for 60 minutes. The cells were pelleted using the microfuge for 1 minute at high speed, and resuspended in 0.5ml of 50mM Tris•Cl, pH 7.4 and 20mM EDTA. 0.05ml of 10% SDS was added, the sample mixed, and incubated at 65°C for 30 minutes. 0.2 ml of 5M potassium acetate pH 5.2 was added and the sample was incubated on ice for 60 minutes. The sample was again spun in a microfuge at high speed for 5 minutes.

The supernatant was transferred to a fresh 1.5ml microfuge tube and 1 volume of isopropanol at room temperature was added. The sample was mixed and allowed to sit at room temperature for 5 minutes, then spun very briefly (10 seconds) in a microfuge at high speed. The supernatant was poured off and the pellet air dried. After resuspending the pellet in 0.3ml of 10mM Tris°Cl, pH 7.4 and 1 mM EDTA, 15$\mu$l of a 1 mg/ml solution of pancreatic RNase was added, and the sample was incubated at 37°C for 30 minutes. 0.03ml of 3M sodium acetate was added, the sample mixed, and 0.2ml of isopropanol added. The sample was spun in a microfuge at high speed to pellet the DNA. The supernatant was then poured off, the pellet dried and resuspended in 0.1-0.3ml of 10mM Tris°Cl, pH 7.4 and 1mM EDTA. (Note: Before using the DNA in a restriction digest, it may be necessary to spin the solution for 15 minutes at high speed in the microfuge to remove any insoluble material which may inhibit the digestion).

## Example V

## Strain Characterization

DNA was prepared from the transformed Pichia cells and from wild type Pichia cells as described in Example IV, and digested with EcoRI. The samples were electrophoresed on 0.8% agarose gels, and Southern blots were performed (Maniatis et al, 1982). The filters were hybridized with an AOX1 specific probe or with a HIS4 specific probe to determine where integration had occurred. The site of integration was determined by comparing the spectrum of hybridization of a given transformant with the wild type strain. Any alteration in the size of the wild type band was evidence of integration at that locus. A summary of the Southern hybridizations and strain characterization is included in Table 5.

Table 5

| Strain | Copy # | Site of integration |
|---|---|---|
| G-GIF211S3 | multi | AOX1 & possibly other sites |
| G-GIF211S5 | multi | AOX1 & possibly other sites |
| G-GIF211S6 | multi | AOX1 & possibly other sites |
| G-GIF211S8 | two | AOX1 & unknown site |
| G-GIF211S4 | one | AOX1 |
| G-GIF211S7 | one | AOX1 |
| G-GIF211S9 | one | AOX1 |
| G-GIF000C6 | multi | AOX1 |
| G-GIF000C5 | one | AOX1 |
| G-GIF000C7 | one | AOX1 |
| G + GIF211S1 | two | HIS4 |
| G + GIF211S7 | two | HIS4 |
| G + GIF211S8 | two | HIS4 |
| G + GIF211S2 | one | AOX1 |
| G + GIF211S9 | one | AOX1 |
| G + GIF211S3 | one | HIS4 |
| G + GIF211S4 | two | AOX1 |
| G + GIF211S5 | two | AOX1 |
| G + GIF000C4 | one | AOX1 |
| G + GIF000C7 | one | AOX1 |
| G + GIF000C8 | one | AOX1 |

## Example VI

18

## Shake Flask Expression Studies

Prior to fermentation, all strains noted in Table I were grown in shake flasks to ascertain expression levels. Routinely, a transformant was seeded into 0.67% yeast nitrogen base containing 2-5% glycerol and grown at 30°C into middle to late log phase. The cells were then collected by centrifugation using a Damon IEC DPR600 clinical centrifuge at 3,000 rpm for 5 minutes. The pellet was washed in sterile water twice, then seeded at a density of 1.0(Mut⁻) or 0.05(Mut⁺) $A_{600}$ units/ml into 0.67% YNB containing 1% methanol and grown for 4-6 days (for Mut⁻ strains) or 30 hours (for Mut⁺ strains) at 30°C with moderate shaking. At various times, aliquots of 50 $A_{600}$ units were removed and stored at -20°C. The shake flask cultures were analyzed for cytoplasmic and secreted IFN-γ by RIA and Western blots as described in Example VIII.

## Example VII

## Fermentation of IFN-γ Strains

The preferred mode of IFN-γ production is the following methanol fed batch fermentation protocol using secretion strain G-GIF211S8 or cytoplasmic strain G-GIF000C6.

Two 10 L runs were performed as follows. Five hundred ml of phosphate-buffered Nitrogen Base (YNB) +2% glycerol in a Fernback Flask were inoculated from a seed culture or a minimal glucose plate of the culture. (Plates may be maintained by monthly passage with no detectable strain deterioration). After one day of shaking at 200 rpm and 30°C, the inoculum was seeded into 7 litres of minimal medium containing 480g glycerol, 40 mg biotin, and 40 ml trace salts solution. The fermentor was maintained at 30°C and pH 5.5 while the culture grew in batch mode until the glycerol was exhausted (18-24 hours). The pH was controlled by the addition of $NH_3$. Glycerol exhaustion was noted by a sharp rise in the dissolved oxygen (or decrease in oxygen uptake rate). A methanol feed was initiated at 18 ml/hr to bring ther fermentor level up to ~0.5% MeOH, and maintained at this level. The flow rate was adjusted, based on the actual MeOH consumption rate. Twenty ml aliquots of trace salts were added at approximately two day intervals to maintain the methanol consumption rate. The level of IFN-γ increased for seven to eight days on the methanol feed. A one liter fermentation (#351; secretion) was scaled down proportionally with the following changes: the initial volume was one liter and the glycerol concentration was 2.0%.

## Example VIII

## Detection and Confirmation of the Presence of IFN-γ

### A. Cell Removal and Broth Concentration

Approximately 7.9 liters were obtained from fermenter run #359 (methanol slow; secretion; G-GIF211S8). Cells were removed by filtration through a 1 micron ceramic cartridge. The cell-free broth was diafiltered on a spiral cartridge (MWCO 3,000) in order to reduce the conductivity and wash out small molecular weight impurities. The volume of the diafiltered broth was not reduced at this time. As needed, the broth was concentrated either with a small spiral cartridge or with an Amicon stirred cell (MWCO 10,000 membrane). When smaller volumes of broth from 11 fermenter runs #350, 351, 377 and 378 were used, cells were removed by centrifugation (10,000 rpm, GSA rotor) and filtration through a 0.45 micron filter. The cell-free broth was concentrated with the small spiral cartridge and stirred cell.

### B. Extraction

Cells were washed and divided into 25 or 100 OD aliquots in 13 X 100 mm glass tubes, and then frozen until extraction. The cells were thawed and washed two times with buffer (0.5M NaCl/0.1% Triton x-100/10mM NaPO₄, pH 7.5). An aliquot of 100 OD of cells was broken with 0.5g of glass beads and 350 μL of breaking buffer (rinse buffer containing 2mM PMSF). The cells were extracted on a multi-sample vortex with four cycles of one minute of vortexing followed by one minute on ice. The liquid was transferred to an eppendorf tube (with a plastic pipet tip), and the beads were rinsed with an additional 350 ul of breaking buffer by vortexing one minute. The rinse was added to the eppendorf tube and centrifuged 15 minutes. The supernatant was stored on ice until the interferon was determined by RIA. An aliquot of 25 OD of cells was broken in 175 ul of breaking buffer with 0.5g glass beads and rinsed with an additional 175 ul.

After extraction, the cell pellet was suspended in 500 ul (100 OD cells) or 350 ul (25 OD cells) of Laemmli buffer [Laemmli, Nature 227:680-685 (1970)], boiled for 5 minutes, and centrifuged for 5 minutes. The supernatants were used for Western analysis ad described below.

## C. Optimized Extraction

The cells were lysed as described above except the tubes were agitated five times, 1-minute duration, alternated with 1 minute on ice and then spun in a clinical centrifuge to reduce foaming. A total breakage volume of 1.4 ml instead of 0.7 ml was used in some cases. Multiple extractions were done by adding 1.0 ml of the respective buffer to the pellet, vortexing briefly, and microfuging. This procedure was repeated up to 3 times. The final pellet was treated as before.

## D. Western Blots

Samples were separated on 0.75 X 6 X 9 cm SDS 15% polyacrylamide gels. The gels were transferred to 0.45μ nitrocellulose paper at 200 mA for 90 minutes. The blots were blocked with GBB (0.25% gelatin/10mM phosphate buffer/0.15M NaCl/0.05% Tween 20/0.2% sodium azide) for 60 minutes. Polyclonal or monoclonal antibody (Interferon Sciences) was diluted 1:1000 in GBB and incubated with the blots overnight at room temperature. The polyclonal antibody was pretreated with lysate from pAO804 cells (1mg/ml) to lower the background from endogenous Pichia proteins. After incubation, the blots were rinsed and incubated in GBB containing 1% BSA for 60 minutes. When monoclonal antibody was used, the blots were incubated with rabbit anti-mouse antibody (1:1000 dilution) for 90 minutes to allow the Protein A to bind. The blots were rinsed and incubated in GBB. Protein A¹²⁵-I (0.1μ Ci/40mlGBB) was added and the incubation continued for 45 minutes. The blots were rinsed and incubated for 45 minutes in GBB followed by a brief treatment with 5% non-fat dry milk to aid in subsequent stripping of the antibody for reuse of the blots. The blots were air dried and exposed to film. Five ng of standard γ-interferon can be detected with the procedure described above.

## E. Expression Levels

The level of IFN-γ was determined by RIA. The values are reported in Tables 6 and 7 and are corrected for cell density.

### Table 6
### Cytoplasmic Expression of IFN-γ
### Strain G-GIF000C6

| Fermentor Run | Mode | Time Hr. | WW g/l | Sp Yield μg/g | Vol Yield mg/l | Sp Prod μg/g-h | Vol Prod (mg/l-h) |
|---|---|---|---|---|---|---|---|
| 385 | FB | 90 | 180 | 560 | 97 | 6.2 | 1.1 |
| 394 | FB | 31 | 105 | 143 | 15 | 4.6 | 0.48 |

## Table 7
## Secretion of IFN-γ

| Strain | Run | Mode | Time hr | WW g/l | Sp Yield γg/g | Vol Yield mg/l | Sp Prod γg/g-h | Vol Prod mg/l-h |
|--------|-----|------|---------|--------|---------------|----------------|----------------|-----------------|
| G-GIF211S6 | 349 | FB | 128 | 228 | 10 | 2.5 | 0.078 | 0.020 |
| G-GIF211S7 | 350 | FB | 110 | 232 | 42 | 9.9 | 0.39 | 0.090 |
| G-GIF211S8 | 351 | FB | 110 | 208 | 78 | 16 | 0.71 | 0.15 |
| G-GIF211S8 | 359 | FB | 160 | 228 | 35 | 8.2 | 0.22 | 0.051 |
| G-GIF211S6 | 363 | FB | 156 | 206 | 12 | 1.4 | 0.075 | 0.009 |

FB = feed batch

F. Endo H Treatment of Broth and Lysates

One hundred microliters of concentrated diafiltered #359 broth or cell lysate was treated with 37 ng of Endo H in the presence of 0.1M sodium acetate, pH 5.2, 1 mM EDTA, +/- 0.1% SDS at 37 degrees for 2 hours. Ten microliters of each sample was subjected to Western blot analysis.

G. Results

i. Secreted IFN-γ:

The immunoreactive IFN found in the broth from these Mut⁻ strains migrates on an SDS-polyacrylamide gel as a cluster of at least three bands with apparent molecular weights of approximately 35 to 38kD. An E. coli produced human γ-IFN standard (Amgen) demonstrates two bands of 17 and 34KD, representing unglycosylated monomer and dimer, respectively. When treated with Endo H as described above the Pichia-produced bands collapse to two distinct bands of 32 and 16kD. The appearance of the 16kD band suggests that some limited proteolysis has occurred. Partial removal of oligosaccharides results in an additional band at 17kD.

ii. Cytoplasmic IFN-γ:

Western blot analyses of lysates from the cytoplasmic strain showed that the IFN consists of a monomer (17kD) and a dimer (34KD), indistinguishable from the standard IFN.

## Claims

1. A process for the enhanced production of IFN-γ comprising
   a) transforming a methylotrophic yeast with at least one vector having at least one expression cassette containing a structural gene for IFN-γ, operably linked to a regulatory region and a 3′ termination sequence; and thereafter
   b) culturing the resulting transformed yeast strain to obtain the production of said IFN-γ.

2. The process of claim 1 wherein said vector is selected from plasmids or linear integrative site-specific vectors.

3. The process of claim 2 wherein said linear integrative site-specific vector contains the following serial arrangement:

a) a first insertable DNA fragment,

b) a marker gene, and at least one expression cassette containing a structural gene for IFN-γ, wherein said structural gene is selected from IFNC-2 and IFN211, operably linked to a regulatory region and a 3′ termination sequence, and

c) a second insertable DNA fragment;

wherein the order of the marker gene and cassette of component (b) may be interchanged.

4. The process of claim 3 wherein the first insertable DNA fragment and the second insertable DNA fragment are derived from the DNA sequence of a gene isolated from Pichia pastoris and selected from AOX1, p40, DHAS and HIS4.

5. The process of claim 3 wherein said expression cassette comprises

a) a regulatory region selected from the group consisting of AOX1, p40, DHAS and HIS4, isolated from Pichia pastoris, acid phosphatase, galactokinase, alcohol dehydrogenase, cytochrome c, alpha-mating factor and glyceraldehyde 3-phosphate dehydrogenase isolated from Saccharomyces cerevisiae operably linked to

b) a structural gene for IFN-γ wherein said structural gene is selected from IFNC-2 and IFN211, operably linked to

c) a 3′ termination sequence from Pichia pastoris selected from the 3′ termination sequences isolated from the AOX1 gene, p40 gene, DHAS gene and HIS4 gene.

6. The process of claim 3 wherein said marker gene is selected from HIS4 and ARG4, isolated from Pichia pastoris, SUC2 isolated from Saccharomyces cerevisiae and G418$^R$ gene of Tn903 and Tn601.

7. The process of claim 3 wherein said vector comprises

a) a first insertable DNA fragment which is about one kilobase of the 5′ AOX1 regulatory region isolated from Pichia pastoris operably linked to

b) a structural gene for IFN-γ wherein said structural gene is selected from IFNC-2 and IFN211, operably linked to

c) the 3′ termination sequence of AOX1 isolated from Pichia pastoris ligated to

d) a marker gene which is HIS4 isolated from Pichia pastoris ligated to

e) a second insertable DNA fragment which is about 0.65 kilobases of the 3′ AOX1 termination sequence.

8. A linear integrative site-specific vector comprising the following serial arrangement

a) a first insertable DNA fragment,

b) a marker gene and at least one expression cassette containing a structural gene for IFN-γ operably linked to a regulatory region and a 3′ termination sequence, and

c) a second insertable DNA fragment;

wherein the order of the marker gene and cassette of component (b) may be interchanged.

9. The vector of claim 8, wherein said vector is as defined in any of claims 4 to 7.

10. Methylotrophic yeast transformed with at least one vector containing at least one expression cassette comprising a regulatory region operably linked to a structural gene for IFN-γ, operably linked to a 3′ termination sequence.

11. The methylotrophic yeast of claim 10 wherein the yeast is Pichia pastoris.

12. The methylotrophic yeast of claim 11 wherein the yeast is Pichia pastoris strain GS115.

13. The methylotrophic yeast of any of claims 10 - 12 wherein said yeast is transformed with at least one linear integrative site-specific vector as defined in any of claims 3 - 9.

14. The methylotrophic yeast of any of claims 10 - 12 wherein said yeast is transformed with more than one copy of said linear integrative site-specific vector.

15. The transformed methylotrophic yeast of claim 10 wherein said yeast is selected from G-GIF211S3, G-GIF211S5, G-GIF211S6, G-GIF211S8, G-GIF211S4, G-GIF211S7, G-GIF211S9, G-GIF000C6, G-GIF000C5, G-GIF000C7, G + GIF211S1, G + GIF211S7, G + GIF211S8, G + GIF211S2, G + GIF211S9, G + GIF211S3, G + GIF211S4, G + GIF211S5, G + GIF000C4, G + GIF000C7 and G + GIF000C8.

FIG. 1

FIG. 2

IFN-$\gamma$ CYTOPLASMIC VECTOR

FIG. 3

*FIG. 4*

IFN-γ  SECRETION VECTOR